# EUROPÄISCHE PATENTSCHRIFT

(11) **EP 3 816 173 B1**
(45) Veröffentlichungstag und Bekanntmachung des Hinweises auf die Patenterteilung: **22.12.2021**
(21) Anmeldenummer: 19205655.4
(22) Anmeldetag: 28.10.2019
(51) Int. Cl.: C07F 9/6574, B01J 31/18, C07B 41/06, C07C 45/50, C07F 15/00

(54) **PHOSPHAZYKLISCHE PHOSPHITE AUS DEM ENOL DES PYRIDOINS UND FUROINS**
PHOSPHACYCLIC PHOSPHITES FROM PYRIDOINE AND FUROIN
PHOSPHITES PHOSPHOCYCLIQUES DE L'ÉNOL DE PYRIDOINE ET DE FUROINE

(43) Veröffentlichungstag der Anmeldung: 05.05.2021
(73) Patentinhaber: Evonik Operations GmbH, 45128 Essen (DE)
(72) Erfinder: FRANKE, Robert, 45772 Marl (DE); BÖRNER, Armin, 18059 Rostock (DE); SELENT, Detlef, 18059 Rostock (DE)
(74) Vertreter: Evonik Patent Association

(56) Entgegenhaltungen:
- EP-A1- 2 003 138

## Beschreibung

Die Erfindung betrifft phosphazyklische Phosphite aus dem Enol des Pyridoins und Furoins.

Phosphorhaltige Verbindungen spielen als Liganden in einer Vielzahl von Reaktionen eine entscheidende Rolle, z.B. in der Hydrierung, in der Hydrocyanierung und auch in der Hydroformylierung.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor P^{III}. Eine gute Übersicht über den Stand der Hydroformylierung von Olefinen findet sich in R. Franke, D. Selent, A. Börner, "Applied Hydroformylation", Chem. Rev., 2012, DOI:10.1021/cr3001803.

In EP 2003138A1 wird ein Verfahren zur Herstellung von Aldehyden beschrieben. Eine Kohlenstoff-Kohlenstoff-Doppelbindung wird mit Kohlenmonoxid und Wasserstoff in Gegenwart eines Bisphosphits und einer Metallverbindung der Gruppen 8 bis 10 umgesetzt.

Das α-Hydroxyketon des Pyridoins und Furoins weist ein Keto-Enol-Gleichgewicht auf:

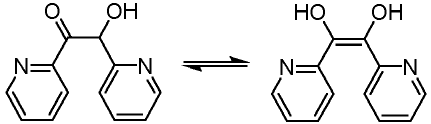

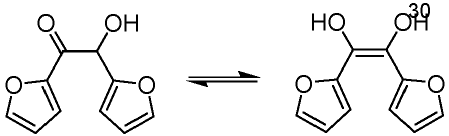

Die technische Aufgabe der Erfindung ist die Bereitstellung neuer Liganden, welche in der Hydroformylierung von Olefinen eine gesteigerte n-Regioselektivität aufweisen.

Die Aufgabe wird gelöst durch eine Verbindung gemäß Anspruch 1.

Verbindung gemäß einer der Strukturen (**I**) oder (**II**): 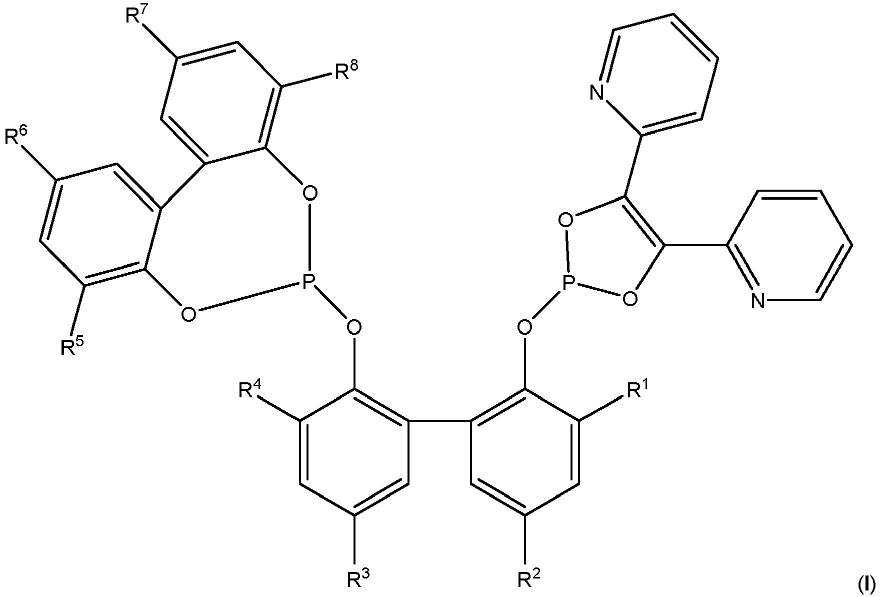 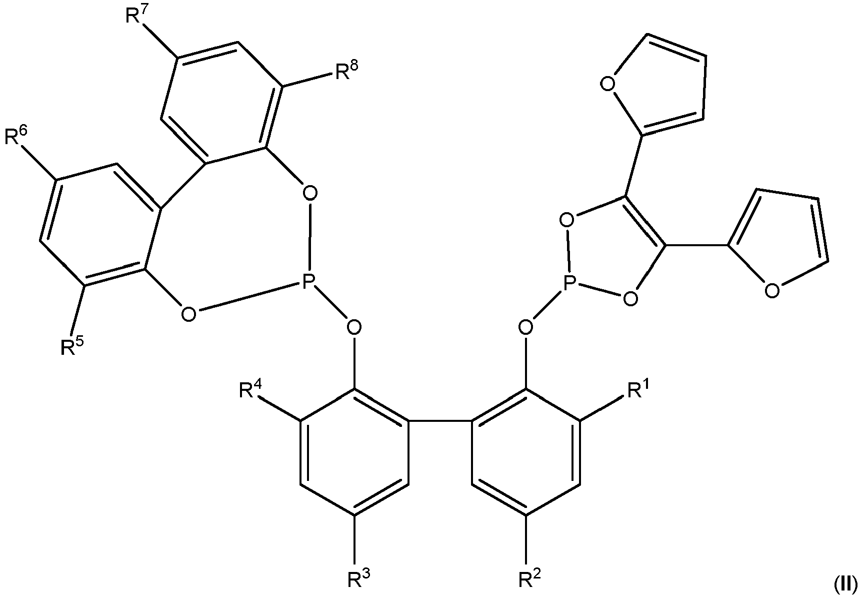 wobei R¹, R², R³, R⁴, R⁵, R⁶, R⁷, R⁶ ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -NO₂, -NH₂, -N[(C₁-C₁₂)-Alkyl]₂.

In einer Ausführungsform sind R¹, R², R³, R⁴ ausgewählt aus: -H, -(C₁-C₁₂)-Alkyl, -O-(C₁-C₁₂)-Alkyl.

In einer Ausführungsform sind R⁵, R⁶, R⁷, R⁸ ausgewählt aus: -H, -(C₁-C₁₂)-Alkyl, -O-(C₁-C₁₂)-Alkyl.

In einer Ausführungsform sind R¹, R², R³, R⁴, R⁵, R⁶, R⁷, R⁸ ausgewählt aus: -H, -(C₁-C₁₂)-Alkyl, -O-(C₁-C₁₂)-Alkyl.

In einer Ausführungsform weist die Verbindung die Struktur (**I**) auf.

In einer Ausführungsform weist die Verbindung die Struktur (1) auf:

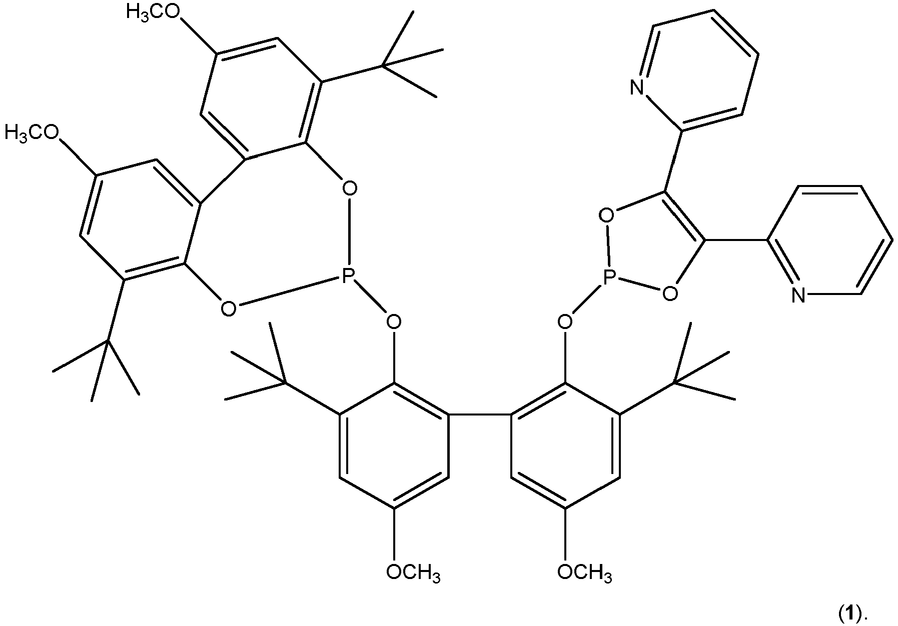

In einer Ausführungsform weist die Verbindung die Struktur (**II**) auf.

In einer Ausführungsform weist die Verbindung die Struktur (2) auf.

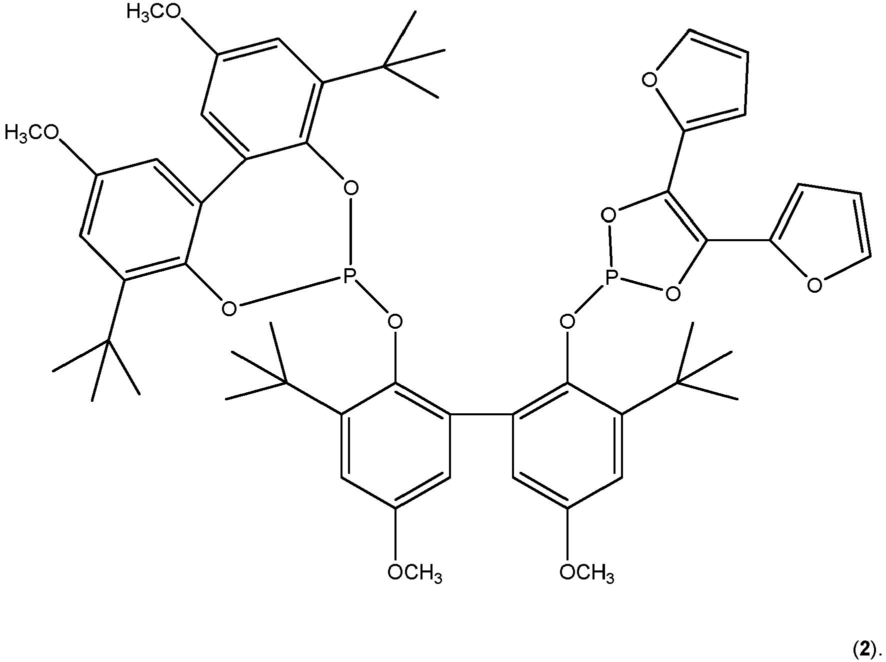

Neben der Verbindung als solche wird auch deren Verwendung zur Katalyse einer Hydroformylierungsreaktion beansprucht.

Verwendung einer zuvor beschriebenen Verbindung in einem Ligand-Metall-Komplex zur Katalyse einer Hydroformylierungsreaktion.

Des Weiteren wird ein Verfahren beansprucht, in welchem die zuvor beschriebene Verbindung als Ligand eingesetzt wird.

Verfahren umfassend die Verfahrensschritte:
a) Vorlegen eines Olefins,
b) Zugabe einer zuvor beschriebenen Verbindung und einer Substanz, welche ein Metall ausgewählt aus: Rh, Ru, Co, Ir aufweist,
c) Zuführen von H₂ und CO,
d) Erwärmen des Reaktionsgemisches aus a) bis c), wobei das Olefin zu einem Aldehyd umgesetzt wird.

In einer bevorzugten Ausführungsform ist das Metall Rh.

Es kann hierbei auch ein Überschuss an Liganden verwendet werden und nicht zwangsläufig jeder Ligand liegt gebunden in Form eines Ligand-Metall-Komplexes vor, sondern ist als freier Ligand im Reaktionsgemisch enthalten.

Die Reaktion wird bei üblichen Bedingungen durchgeführt.

Bevorzugt sind eine Temperatur von 80 °C bis 160 °C und ein Druck von 10 bis 70 bar. Besonders bevorzugt sind eine Temperatur von 100 °C bis 140 °C und ein Druck von 10 bis 60 bar.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1-Buten, 2-Buten, 1- oder 2-Penten, 2-Methyl-1-buten, 2-Methyl-2-buten, 3-Methyl-1-buten, 1-, 2- oder 3,-Hexen, das bei der Dimerisierung von Propen anfallende C₆-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende C₈-Olefingemisch (Di-n-buten, Di-iso-buten), Nonene, 2- oder 3-Methyloctene, das bei der Trimerisierung von Propen anfallende C₉-Olefingemisch (Tripropen), Decene, 2-Ethyl-1-octen, Dodecene, das bei derTetramerisierung von Propen oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabuten) sowie durch Cooligomerisierung von Olefinen mit unterschiedlicher Anzahl von Kohlenstoffatomen (bevorzugt 2 bis 4) hergestellte Olefingemische.

Mit dem erfindungsgemäßen Verfahren können unter Verwendung der erfindungsgemäßen Liganden α-Olefine, endständig verzweigte, innenständige und innenständig verzweigte Olefine hydroformyliert werden.

Im Folgenden soll die Erfindung anhand von Ausführungsbeispielen näher erläutert werden.

### Arbeitsvorschriften

### Allgemeine Analytik

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet.

Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR³¹P = SR¹H * (BF³¹P / BF¹H) = SR¹H * 0,4048.

### Synthese 2-Chlor-4,5-diphenyl-1,3,2-dioxaphosphol. (Intermediat A)

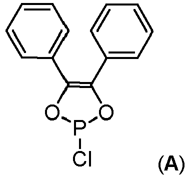

Zu einer Lösung von Benzoin (3 g; 14,134 mmol) in THF (60 ml) wird bei -40 °C unter starkem Rühren zunächst Phosphortrichlorid (3,083 g; 22,692 mmol) und anschließend Triethylamin (5,9 ml) getropft. Man setzt noch THF zu (10 ml) und rührt über Nacht, filtriert und engt das Filtrat im Vakuum ein. Der erhaltene ölige Rückstand wird 4 h bei 50 °C/ 1 mbar getrocknet und wie erhalten zur weiteren Synthese eingesetzt. Ausbeute: 3,494 g (12,629 mmol; 89 %).
³¹P-NMR (CD₂Cl₂): 169,1 (s) ppm.
¹H-NMR (CD₂Cl₂): 7,41 (m, 6H); 7,60 (m, 4 H) ppm.

### Synthese 2,2'-(2-((3,3'-Di-tert.-butyl-2'-((4,8-di-tert.-butyl-2,10-dimethoxydibenzo[d,f][1,3,2] dioxaphosphepin-6-yl)oxy)-5,5'-dimethoxy-[1,1'-biphenyl]-2-yl)oxy)-1,3,2-dioxaphosphole-4,5-diyl)dipyridine (1)

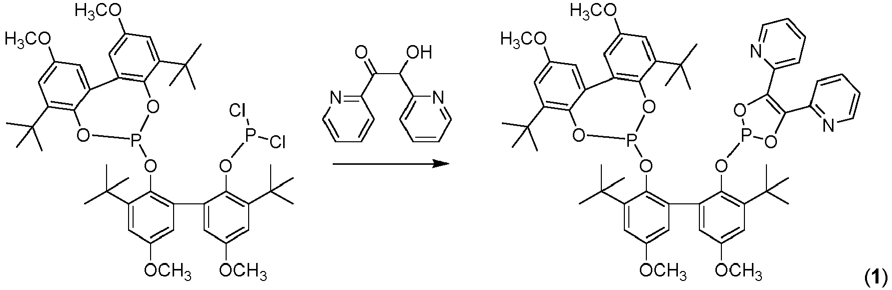

Zu einer Lösung von 4,8-Di-*tert*.-butyl-6-((3,3'-di-*tert*.-butyl-2'-((dichlorophosphanyl)oxy)-5,5'-dimethoxy-[1,1'-biphenyl]-2-yl)oxy)-2,10-dimethoxydibenzo[*d*,*f*][1,3,2]dioxaphosphepin (0,803 g; 0,949 mmol) in Toluol (4,5 ml) wird bei Raumtemperatur eine Mischung aus Pyridoin (0,203 g; 0,961 mmol) und Triethylamin (7,5 ml) in Toluol (5 ml) getropft. Man lässt über Nacht rühren, filtriert, und engt das Filtrat im Vakuum zur Trockne ein. Der erhaltene gelbe Feststoff wird mit Heptan (10 ml) verrührt. Man filtriert, wäscht mit Heptan und trocknet den isolierten Feststoff 3 h bei 50 °C/ 0,1 mbar. Säulenchromatografische Reinigung (CH₂Cl₂, R*_{f}*= 0,74) ergibt 0,679 g (0,688 mmol; 70%). Elementaranalyse (berechnet für C₅₆H₆₄O₁₀P₂N₂, 987,07 g/mol): C, 68,12 (68,14); H, 6,42 (6,54); P, 6,11 (6,28); N, 2,86 (2,83)%.
ESI-TOF HRMS, *m*/*z=* 987,41210; [M⁺]; berechnet *m*/*z*= 987,41149.
³¹P-NMR (CD₂Cl₂): 131,8 (d, Jpp= 18,0 Hz); 140,4 (s, br) ppm.
¹H-NMR (CD₂Cl₂): 1,13 (s, 9H); 1,29 (s, 9H); 1,40 (s, 9H); 1,46 (s, 9H); 3,81 (s, 3H); 3,82 (s, 3H); 3,83 (s, 3H); 3,85 (s, 3H); 6,69 (d, *J*_{HH}= 3,0 Hz; 1H); 6,72 (d, *J*_{HH}= 3,0 Hz; 1H); 6,80 (m, 1H); 6,90 (d, *J*_{HH}= 3,2 Hz; 1H); 6,92 (d, *J*_{HH}= 3,2 Hz; 1H); 6,97 (d, *J*_{HH}= 3,3 Hz; 1H); 7,03 (d, *J*_{HH}= 3,1 Hz; 1H); 7,11 (d, *J*_{HH}= 3,3 Hz; 1H); 7,25-7,30 (m, 2H); 7,59 (dt, *J*_{HH}= 8,0/1,1 Hz; 1H); 7,73-7,84 (m, 3H); 8,59 (m, 2H) ppm.

### Synthese 4,8-Di-tert.-butyl-6-((3,3'-di-tert.-butyl-2'-((4,5-di(furan-2-yl)-1,3,2-dioxaphosphol-2-yl)oxy)-5,5'-dimethoxy-[1,1'-biphenyl]-2-yl)oxy)-2,10-dimethoxydibenzo[d,fl[1,3,2]dioxa-phosphepin (2)

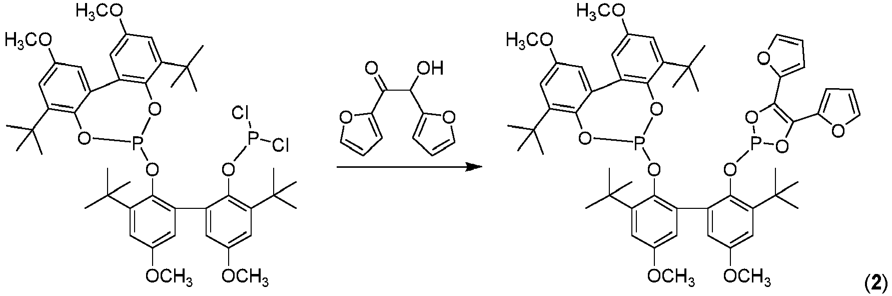

Zu einer Lösung von 4,8-Di-*tert*.-butyl-6-((3,3'-di-*tert*.-butyl-2'-((dichlorophosphanyl)oxy)-5,5'-dimethoxy-[1,1'-biphenyl]-2-yl)oxy)-2,10-dimethoxydibenzo[*d,f*][1,3,2]dioxaphosphepin (0,802 g; 0,948 mmol) in Toluol (4 ml) wird bei Raumtemperatur eine Mischung aus Furoin (0,182 g; 0,948 mmol) und Triethylamin (0,53 ml) in Toluol (5 ml) gegeben. Man lässt über Nacht rühren, filtriert, und engt das Filtrat im Vakuum zur Trockne ein. Das erhaltene hoch viskose Produkt wird 2 h mit Heptan (11 ml) verrührt. Man filtriert, wäscht den Feststoff mit Heptan und trocknet 3 h bei 50 °C/ 0,1 mbar. Ausbeute: 0,707 g (0,733 mmol; 77%).

Elementaranalyse (berechnet für C₅₄H₆₂O₁₂P₂, 965,02 g/mol): C, 66,91 (67,21); H, 6,49 (6,48); P, 6,34 (6,42)%.
ESI-TOF HRMS, *m*/*z=* 965,3792; [M⁺+H]; berechnet: 965,3795.
³¹P-NMR (CD₂Cl₂): 131,0 (d, *J*PP= 19,4 Hz); 140,4 (s, br) ppm.
¹H-NMR (CD₂Cl₂): 1,17 (s, 9H); 1,24 (s, 9H); 1,39 (s, 9H); 1,43 (s, 9H); 3,81 (s, 3H); 3,83 (s, 3H); 3,84 (s, 3H); 3,85 (s, 3H); 6,54 (dd, *J*_{HH}= 3,5/3,5 Hz; 1H); 6,58 (dd, *J*_{HH}= 3,5/3,5 Hz; 1H); 6,65 (d, *J*_{HH}= 3,4 Hz; 1H); 6,67 (d, *J*_{HH}= 3,1 Hz; 1H); 6,71 (d, *J*_{HH}= 3,1 Hz; 1H); 6,76 (m, 1H); 6,83 (d, *J*_{HH}= 3,4 Hz; 1H); 6,90 (m, 2H); 6,96 (d, *J*_{HH}= 3,2 Hz; 1H); 7,02 (d, *J*_{HH}= 3,2 Hz; 1H); 7,11 (d, *J*_{HH}= 3,3 Hz; 1H); 7,54 (m, 1H); 7,58 (m, 1H) ppm.

### Synthese 2,2'-((3.3'-Di-tert.-butyl-5.5'-dimethoxy-[1.1'-biphenyl]-2,2'-diyl)bis(oxy))bis(4,5-diphenyl-1,3,2-dioxaphosphol) (3) (Vergleichsligand)

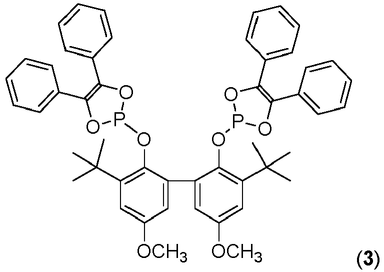

Zu einer auf 3 °C gekühlten Mischung von 3,3'-Di-tert.-butyl-5,5'-dimethoxy-[1,1'-biphenyl]-2,2'-diol (1,070 g; 2,984 mmol) und Pyridin (0,53 ml) in THF (15 ml) wird unter Rühren eine auf 3 °C gekühlte Lösung von Intermediat (A) (1,651 g; 5,969 mmol) in THF (24 ml) getropft. Man lässt anschließend auf Raumtemperatur erwärmen, rührt über Nacht und filtriert. Das Filtrat wird im Vakuum eingeengt und der Rückstand säulenchromatografisch aufgearbeitet (Toluol/Hexan=1:1, R*_{f}*= 0,25). Ausbeute: 1,446 g (1,724 mmol; 58 %).

Elementaranalyse (berechnet für C₅₀H₄₈O₈P₂=838,87 g/mol): C=71,88 (71,59); H=5,59 (5,77); P=7,25 (7,38) %.
³¹P-NMR (CD₂Cl₂): 129,8 (s) ppm.
¹H-NMR (CD₂Cl₂): 1,39 (s, 18H); 3,72 (s, 6H); 6,74 (m, 2H); 7,04 (d, *J*_{HH}=3,2 Hz); 7,30-7,35 (m, 6H); 7,40-7,44 (m, 4H) ppm.
ESI MS: m/z=861,27229; [M⁺+Na]; berechnet m/z=861,27166

### Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol in einem Pure Solv. MD-7 System gereinigt und unter Argon aufbewahrt. Das als Substrat eingesetzte Olefin, 2-Penten (Aldrich), wurden über Natrium am Rückfluss erhitzt und unter Argon destilliert. Im Autoklaven wurden unter Argonatmosphäre Lösungen der Katalysatorvorstufe und des Liganden, jeweils in Toluol, gemischt. Als Katalysatorvorstufe kam [(acac)Rh(COD)] (Umicore, acac=Acetylacetonat-Anion; COD=1,5-Cyclooctadien), zum Einsatz. Der Autoklav wurde bei einem Gesamtgasdruck (Synthesegas: Linde; H2 (99,999%) : CO (99,997%) = 1:1) von 12 bar für den Enddruck von 20 bar unter Rühren (1500 U/min) aufgeheizt. Nach Erreichen der Reaktionstemperatur wurde der Synthesegasdruck auf 19,5 bar erhöht und das Olefin mit einem in der Druckpipette eingestellten Überdruck von ca. 3 bar in den Autoklaven gepresst. Die Reaktion wurde bei konstantem Druck, 20 bar (Nachdruckregler der Fa. Bronkhorst, NL), über 4h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 10 ml Pentan verdünnt und gaschromatographisch analysiert: HP 5890 Series II plus, PONA, 50 m x 0,2 mm x 0,5 µm.

### Ergebnisse der Katalyseversuche

[Rh]: 100 ppm, Rh:L = 1:2, p: 20 bar, T: 120 °C; t: 4 h

**Tabelle 1: Hydroformylierung 2-Penten**

| Eintrag | Ligand | Regioselektivität in % |
|---|---|---|
| 1 | **1*** | 69,0 |
| 2 | **2*** | 70,5 |
| 3 | **3** | 62,9 |

| | | |
|---|---|---|
| * erfindungsgemäße Verbindung | | |

Definition der Selektivität:
Bei der Hydroformylierung gibt es die n/iso-Selektivitäten: das Verhältnis von linearem Aldehyd (= n) zu verzweigtem Aldehyd (= iso). Hierbei bedeutet die Regioselektivität bezüglich des n-Aldehyden, dass diese Menge an linearem Produkt gebildet wurde. Die restlichen Prozente entsprechen dann dem verzweigten Isomer. Bei einer Regioselektivität von 50% entstehen also n-Aldehyd und iso-Aldehyd zu gleichen Teilen.

Mit den erfindungsgemäßen Verbindungen (**1**) und (**2**) konnte jeweils eine gegenüber dem Vergleichsliganden (**3**) gesteigerte Regioselektivität erzielt werden.

Die durchgeführten Versuche belegen, dass die gestellte Aufgabe durch die erfindungsgemäßen Verbindungen gelöst wird.

## Patentansprüche

1. Verbindung gemäß einer der Strukturen (**I**) oder (**II**): 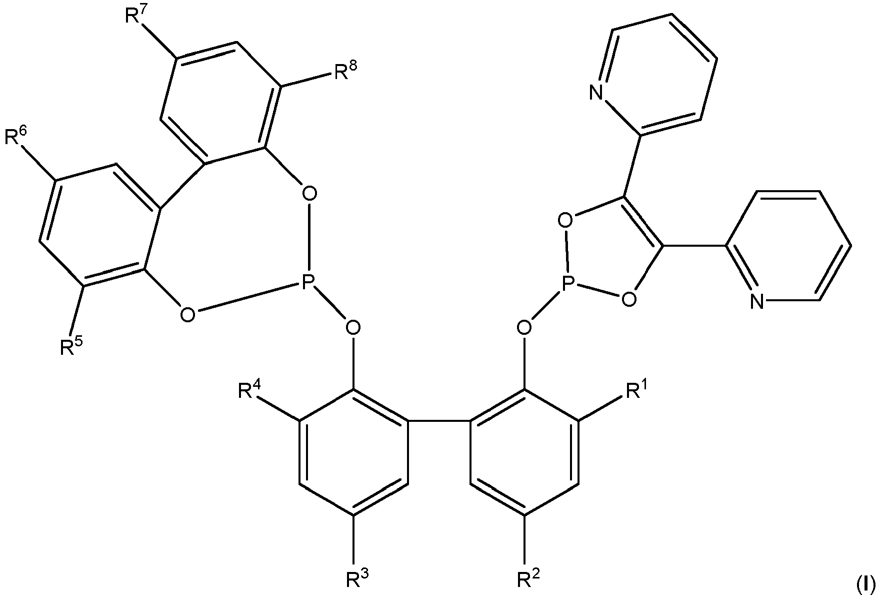 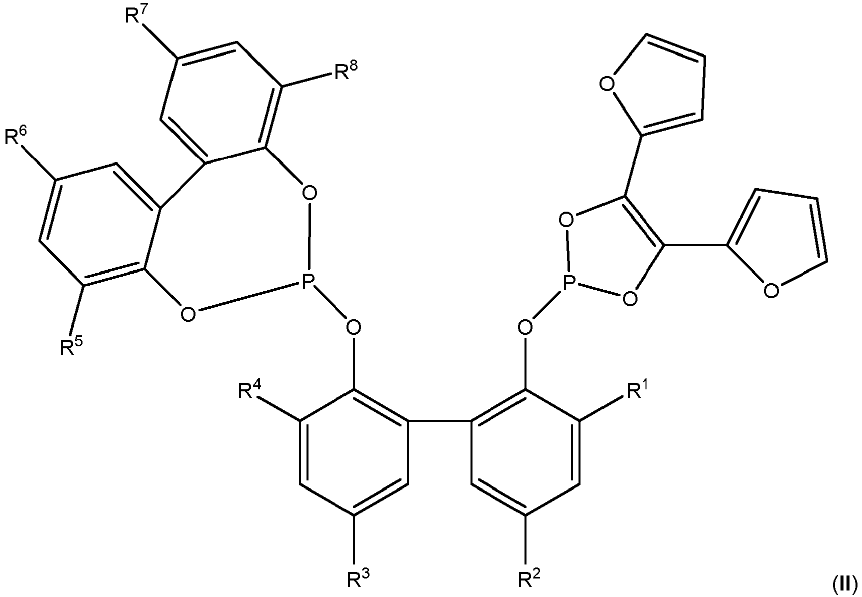 wobei R¹, R², R³, R⁴, R⁵, R⁶, R⁷, R⁸ ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -NO₂, -NH₂, -N[(C₁-C₁₂)-Alkyl]₂.

2. Verbindung nach Anspruch 1,
wobei R¹, R², R³, R⁴ ausgewählt sind aus: -H, -(C₁-C₁₂)-Alkyl, -O-(C₁-C₁₂)-Alkyl.

3. Verbindung nach einem der Ansprüche 1 oder 2,
wobei R⁵, R⁶, R⁷, R⁸ ausgewählt sind aus: -H, -(C₁-C₁₂)-Alkyl, -O-(C₁-C₁₂)-Alkyl.

4. Verbindung nach einem der Ansprüche 1 bis 3,
wobei R¹, R², R³, R⁴, R⁵, R⁶, R⁷, R⁸ ausgewählt sind aus: -H, -(C₁-C₁₂)-Alkyl, -O-(C₁-C₁₂)-Alkyl.

5. Verbindung nach einem der Ansprüche 1 bis 4,
wobei die Verbindung die Struktur (**I**) aufweist.

6. Verbindung nach Anspruch 5,
wobei die Verbindung die Struktur (**1**) aufweist:

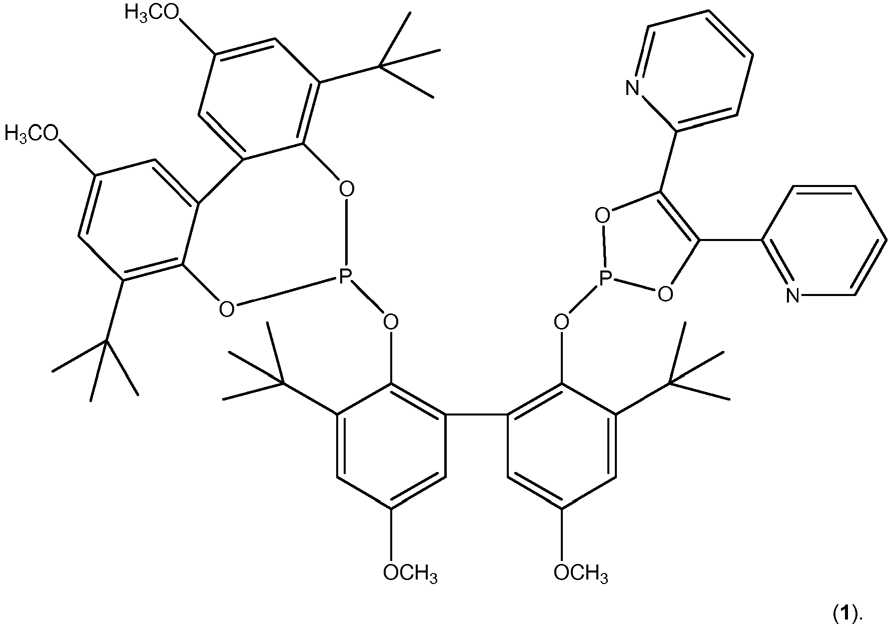

7. Verbindung nach einem der Ansprüche 1 bis 4, wobei die Verbindung die Struktur (**II**) aufweist.

8. Verbindung nach Anspruch 7,
wobei die Verbindung die Struktur (2) aufweist.

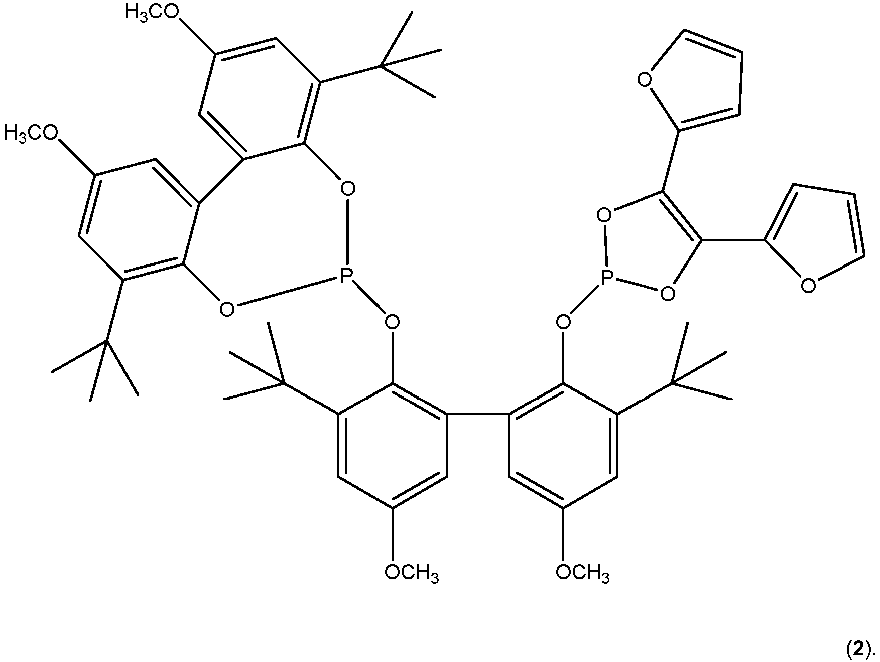

9. Verwendung einer Verbindung nach einem der Ansprüche 1 bis 8,
in einem Ligand-Metall-Komplex zur Katalyse einer Hydroformylierungsreaktion.

10. Verfahren umfassend die Verfahrensschritte:
a) Vorlegen eines Olefins,
b) Zugabe einer Verbindung nach einem der Ansprüche 1 bis 8 und einer Substanz, welche ein Metall ausgewählt aus: Rh, Ru, Co, Ir aufweist,
c) Zuführen von H₂ und CO,
d) Erwärmen des Reaktionsgemisches aus a) bis c), wobei das Olefin zu einem Aldehyd umgesetzt wird.

## Claims

1. Compound according to one of the structures (**I**) or (**II**) : 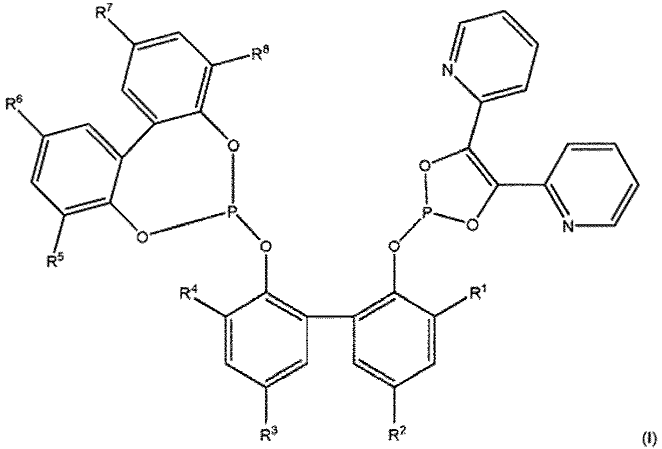 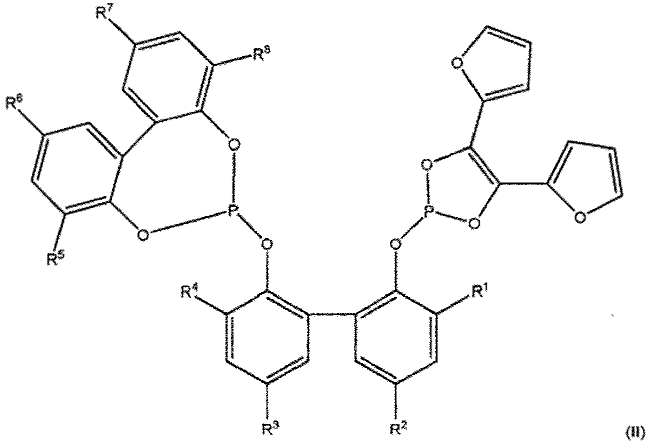 where R¹, R², R³, R⁴, R⁵, R⁶, R⁷, R⁸ are selected from: -H, -(C₁-C₁₂)-alkyl, -O-(C₁-C₁₂)-alkyl, -(C₆-C₂₀)-aryl, -O-(C₆-C₂₀)-aryl, -NO₂, -NH₂, -N[(C₁-C₁₂)-alkyl]₂.

2. Compound according to Claim 1,
where R¹, R², R³, R⁴ are selected from: -H, -(C₁-C₁₂)-alkyl, -O-(C₁-C₁₂)-alkyl.

3. Compound according to either of Claims 1 or 2, where R⁵, R⁶, R⁷, R⁸ are selected from: -H, -(C₁-C₁₂)-alkyl, -O-(C₁-C₁₂)-alkyl.

4. Compound according to any of Claims 1 to 3,
where R¹, R², R³, R⁴, R⁵, R⁶, R⁷, R⁸ are selected from: -H, -(C₁-C₁₂)-alkyl, -O-(C₁-C₁₂)-alkyl.

5. Compound according to any of Claims 1 to 4, wherein the compound has the structure (I).

6. Compound according to Claim 5,
wherein the compound has the structure (1):

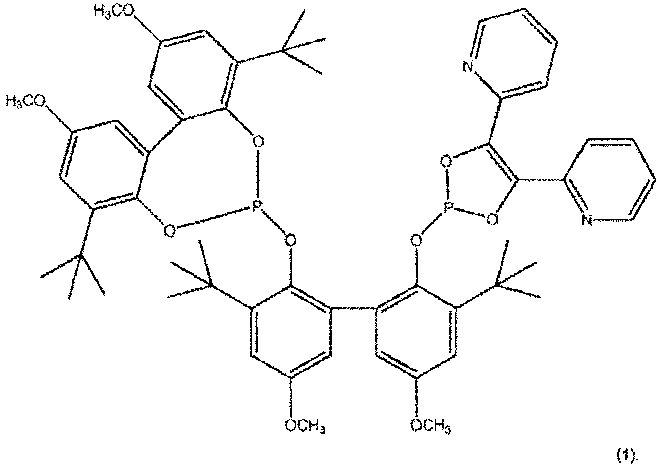

7. Compound according to any of Claims 1 to 4, wherein the compound has the structure (II).

8. Compound according to Claim 7,
wherein the compound has the structure (2)

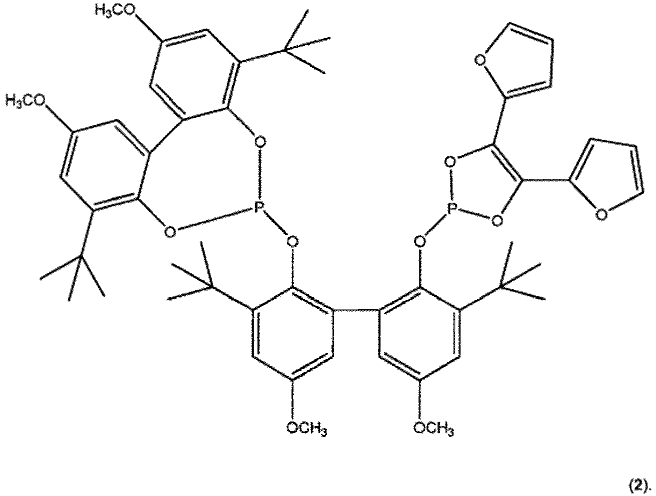

9. Use of a compound according to any of Claims 1 to 8 in a ligand-metal complex for catalysis of a hydroformylation reaction.

10. Process comprising the process steps of:
a) charging an olefin,
b) adding a compound according to any of Claims 1 to 8 and a substance comprising a metal selected from : Rh, Ru, Co, Ir,
c) feeding in H₂ and CO,
d) heating the reaction mixture of a) to c), wherein the olefin is converted to an aldehyde.

## Revendications

1. Composé selon l'une quelconque des structures (I) ou (II) : 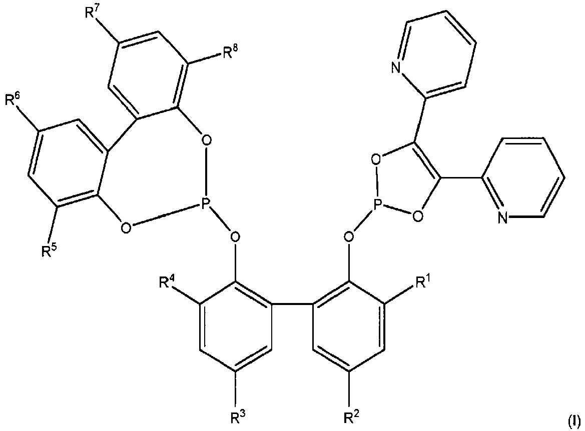 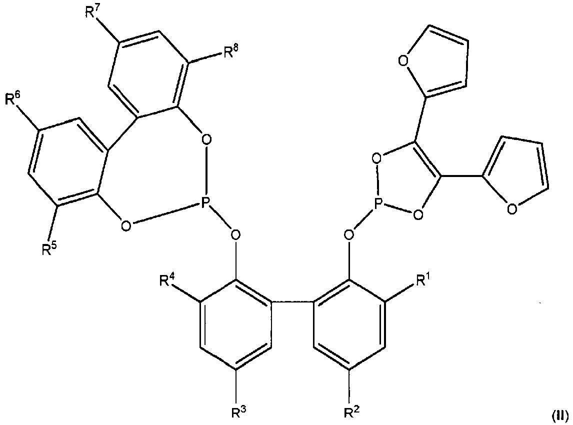 R¹, R², R³, R⁴, R⁵, R⁶, R⁷, R⁸ étant choisis parmi : -H, -(C₁-C₁₂)-alkyle, -O-(C₁-C₁₂)-alkyle, -(C₆-C₂₀)-aryle, -O-(C₆-C₂₀)-aryle, -NO₂, -NH₂, -N[(C₁-C₁₂)-alkyle]₂.

2. Composé selon la revendication 1,
R¹, R², R³, R⁴ étant choisis parmi : -H, -(C₁-C₁₂)-alkyle, -O-(C₁-C₁₂)-alkyle.

3. Composé selon l'une quelconque des revendications 1 ou 2,
R⁵, R⁶, R⁷, R⁸ étant choisis parmi : -H, -(C₁-C₁₂)-alkyle, -O-(C₁-C₁₂)-alkyle.

4. Composé selon l'une quelconque des revendications 1 à 3,
R¹, R², R³, R⁴, R⁵, R⁶, R⁷, R⁸ étant choisis parmi : -H, -(C₁-C₁₂)-alkyle, -O-(C₁-C₁₂)-alkyle.

5. Composé selon l'une quelconque des revendications 1 à 4,
le composé présentant la structure (I).

6. Composé selon la revendication 5,
le composé présentant la structure (1).

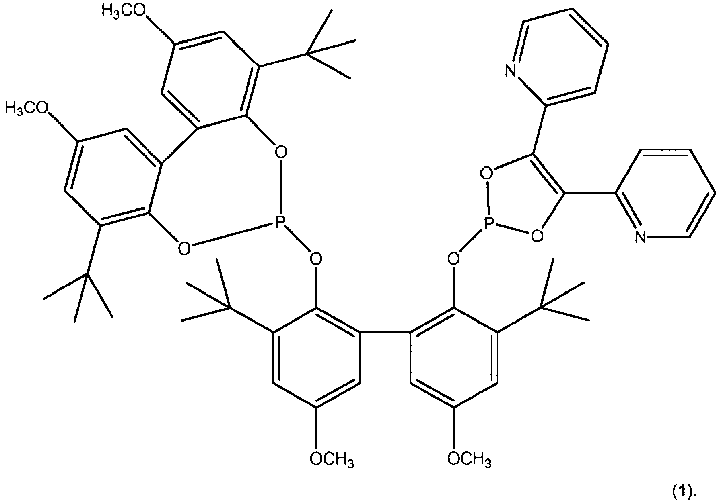

7. Composé selon l'une quelconque des revendications 1 à 4,
le composé présentant la structure (II).

8. Composé selon la revendication 7,
le composé présentant la structure (2).

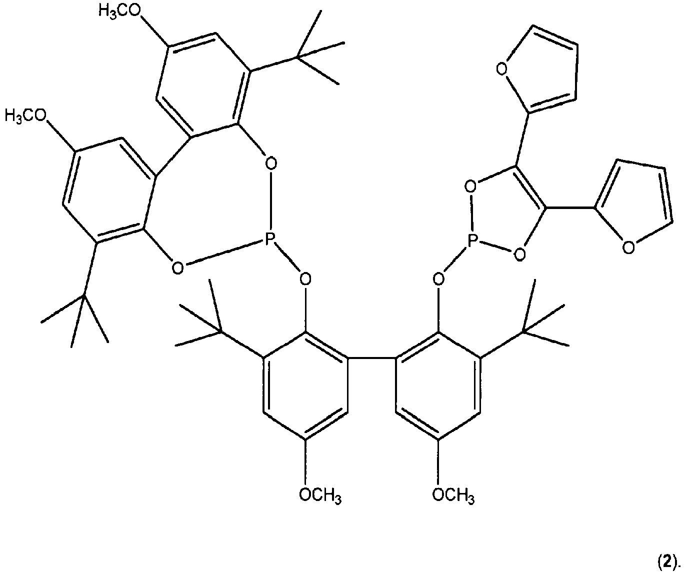

9. Utilisation d'un composé selon l'une quelconque des revendications 1 à 8 dans un complexe ligand-métal pour la catalyse d'une réaction d'hydroformylation.

10. Procédé comprenant les étapes de procédé :
a) disposition préalable d'une oléfine,
b) addition d'un composé selon l'une quelconque des revendications 1 à 8 et d'une substance qui présente un métal choisi parmi : Rh, Ru, Co, Ir,
c) introduction de H₂ et de CO,
d) chauffage du mélange réactionnel de a) à c), l'oléfine étant transformée en aldéhyde.
